(19) Europäisches Patentamt
European Patent Office
Office européen des brevets

(11) Veröffentlichungsnummer: **0 139 933**
**A1**

(12) # EUROPÄISCHE PATENTANMELDUNG

(21) Anmeldenummer: 84109441.0

(22) Anmeldetag: 08.08.84

(51) Int. Cl.⁴: C 07 C 103/70

(30) Priorität: 23.08.83 DE 3330326

(43) Veröffentlichungstag der Anmeldung: 08.05.85 Patentblatt 85/19

(84) Benannte Vertragsstaaten: BE DE FR GB NL

(71) Anmelder: DEUTSCHE TEXACO AKTIENGESELLSCHAFT
Überseering 40
D-2000 Hamburg 60(DE)

(72) Erfinder: Humbert, Heiko, Dr.
Riepenhauserweg 5
D-2100 Hamburg 90(DE)

(72) Erfinder: Laping, Karlheinz, Dr.
Wehmstrasse 5
D-4130 Moers 2(DE)

(74) Vertreter: Schupfner, Gerhard D.
Müller, Schupfner & Gauger Karlstrasse 5 Postfach 14 27
D-2110 Buchholz/Nordheide(DE)

(54) **Herstellung von kristallinen Acryl- und Methacrylamido-alkyltrialkylammoniumcloriden.**

(57) Kristalline Acryl- und Methacrylamidoalkyltrialkylammoniumchloride werden durch Umsetzung der entsprechenden Dialkylaminoalkylacrylamide bzw.-methacrylamide mit Alkylchlorid in Aceton, MTBE oder auch Cyclohexan erhalten.

EP 0 139 933 A1

Herstellung von kristallinen Acryl- und Methacrylamidoalkyltrialkylammoniumchloriden

Die vorliegende Erfindung bezieht sich auf die Herstellung von kristallinen Acryl- und Methacrylamidoalkyltrialkylammoniumchloriden. Polymere auf Basis von Acryl- und Methacrylamidoalkyltrialkylammoniumchlorid-Monomeren sind Mittel für die Feststoff/Flüssig-Trennung.
Methacrylamidopropyltrimethylammoniumchlorid ist ein wichtiges Monomer zur Herstellung von wasserlöslichen kationischen Polymeren. Diese finden vor allem Anwendung als Flockungs- und Retentionshilfsmittel bei der Papierherstellung. Die aus Acryl- und Methacrylamidoalkyltrimethylammoniumchloriden hergestellten Polymeren sind insbesondere ausgezeichnete Flockungs- und Entwässerungsmittel für die Abwasseraufbereitung.

Bei den bekannten Methoden wird die Quarternisierung mit Methylchlorid in wäßriger Lösung durchgeführt. Man erhält die Ammoniumchloride nicht kristallin sondern in Wasser gelöst. Als wesentlicher Nachteil dieser Vorgehensweise sind zu nennen:

1. Die Umsetzung muß in der Regel bei höherer Temperatur, etwa ab 60°C, durchgeführt werden.
2. Die Gegenwart von Wasser führt zu unerwünschten Hydrolyseprodukten.
3. Im Ausgangsmonomer vorhandene Verunreinigungen müssen, soweit überhaupt möglich, in nachfolgenden Schritten abgetrennt werden, etwa gemäß US-PS 3 907 891 mit Aktivkohle.

Gemäß den deutschen Offenlegungsschriften 28 56 383 und 29 11 642 werden entsprechende Verbindungen durch Quarternierung der Amide mit Dimethylsulfat in wäßriger Phase hergestellt und 60 % wäßrige Lösungen des quartären Produkts erhalten. Für diese Produkte

...

gelten die oben angegebenen Nachteile 2. und 3. in gleicher Weise.

Es bestand daher die Aufgabe, Acryl- und Methacrylamidoalkyltrialkylammoniumchloride in reiner kristalliner Form herzustellen.

In Lösung der gestellten Aufgabe wird ein Verfahren zur Herstellung von kristallinen Acryl- und Methacrylamidoalkyltrialkylammoniumchloriden der allgemeinen Formel

$$CH_2 = \underset{R_1}{\underset{|}{C}} - \underset{O}{\underset{\|}{C}} - NH - R_2 - N^{\oplus}(R)_3 \quad Cl^{\ominus}$$

bereitgestellt,
worin R, welches gleich oder verschieden sein kann, eine Alkylgruppe mit 1 bis 3 C-Atomen, bevorzugt Methyl, $R_1$ Wasserstoff oder die Methylgruppe und $R_2$ die zweiwertige Äthylen-, Propylen- oder 2,2-Dimethylpropylen-Gruppe bedeuten, durch Umsetzung von

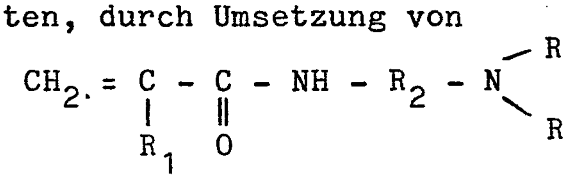

mit RCl,
worin R, $R_1$ und $R_2$ die o.g. Bedeutung besitzen, das dadurch gekennzeichnet ist, daß man die Umsetzung in Aceton, Methyltert.butyläther (MTBE) oder Cyclohexan durchführt und das sich abscheidende kristalline Produkt abfiltriert.

Die vorliegende Erfindung beschreibt einen neuen Prozeß zur Herstellung von Acryl- und Methylacrylamidoalkyltrialkylammoniumchloriden in kristalliner Form. Die Produkte zeichnen sich aufgrund des Herstellungsprozesses durch eine hohe Reinheit aus und können deshalb ohne weitere Reinigung weiterverarbeitet werden.

Bei dem erfindungsgemäßen Verfahrens wird die Quarternisierung der N-substituierten Acryl- und Methacrylamide mit Alkylchlorid bevorzugt in Aceton durchgeführt. Die entstehenden Ammoniumchloride sind unlöslich u. fallen im Verlauf der Umsetzung praktisch vollständig aus.

Die Kristalle werden von der Mutterlauge abgetrennt und getrocknet. Hierbei bleiben die in der Mehrzahl im Lösungsmittel löslichen Verunreinigungen, so vor allem das aufgrund seiner Vernetzereigenschaften störende und in DMAPMA und DMAPAA vorhandene Allylmethacrylamid (AMA) bzw. Allylacrylamid (AAA), in der Mutterlauge. Ein weiterer Vorteil ist darin zu sehen, daß der im substituierten Acryl- bzw. Methacrylamid vorhandene Inhibitor, meist Hydrochinonmonomethyläther (MEHQ), Di-tert-butylkresol und ähnliches, ebenfalls abgetrennt werden und das Produkt mit einem neuen Inhibitor nach Wahl versetzt werden kann.

Die kristallinen Produkte können in verschiedener Weise weiterverarbeitet werden. Wenn man sie in Wasser auflöst, erhält man klare, farblose Lösungen.

Folgende Verbindungen können z.B. erfindungsgemäß hergestellt werden:

Methacrylamidopropyltrimethylammoniumchlorid (MAPTAC)

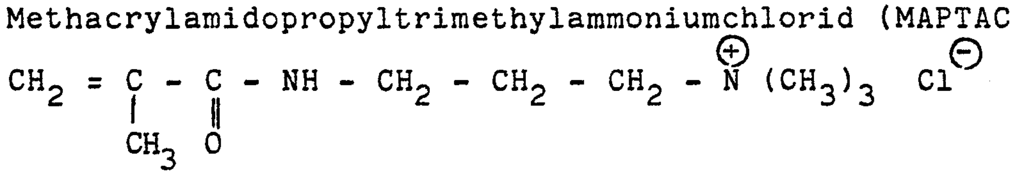

ausgehend vom Dimethylaminopropylmethacrylamid (DMAPMA)

Acrylamidopropyltrimethylammoniumchlorid (APTAC)

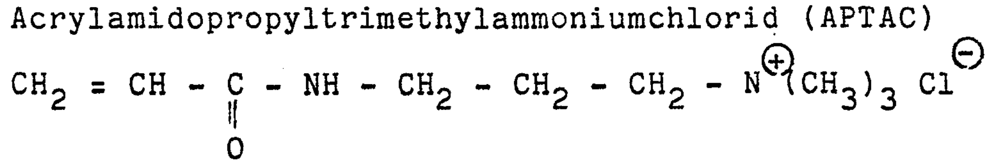

ausgehend vom Dimethylaminopropylacrylamid (DMAPAA)

Acrylamidoneopentyltrimethylammoniumchlorid (ANTAC)

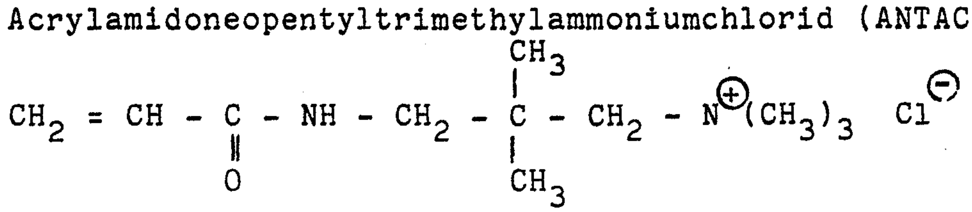

ausgehend vom N,N,2,2-Tetramethylaminopropylacrylamid (TEMAPA).

...

Von den untersuchten Monomeren zeigt DMAPAA mit Methylchlorid die höchste Reaktivität. Während DMAPAA bei Raumtemperatur und DMAPMA am günstigsten bei 40 bis 50°C umgesetzt wird, muß zur Quarternisierung von TEMAPA die Temperatur auf 80 bis 90°C eingestellt werden.

Bei den beiden erstgenannten Monomeren kann in einem offenen Rührwerk gearbeitet werden, da das Methylchlorid sofort absorbiert und umgesetzt wird. Bei TEMAPA läßt man die Quarternisierung unter autogenem Druck ablaufen.

Die Reaktionsgeschwindigkeit ist bei allen Monomeren in Aceton am größten. In Cyclohexan und MTBE läßt sich bei dem reaktiven DMAPAA oder bei höherer Temperatur eine einem technischen Verfahren angemessene Reaktionsgeschwindigkeit erreichen.

Hinsichtlich der Reihenfolge des Zusammengebens der Reaktionskomponenten bieten sich zwei Verfahrensweisen an. Es kann in eine Lösungsmittel-Monomer-Mischung Alkylchlorid gasförmig eingeleitet werden. Es ist aber auch möglich, eine Lösung von Alkylchlorid im Lösungsmittel vorzulegen und das Monomer langsam zuzugeben. Bei der letzteren Verfahrensweise wird in einem geschlossenen Gefäß gearbeitet, um ein Ausgasen des Alkylchlorids zu verhindern.

Die folgenden Beispiele erläutern die Erfindung:

<u>Beispiel 1</u>

Herstellung von Acrylamidopropyltrimethylammoniumchlorid

936 g (6 Mol) DMAPAA, welches mit 1000 ppm Di-tert.-butylkresol inhibiert ist, wurden in 5000 ml Aceton gelöst. 354 g (7 Mol) Methylchlorid wurden gasförmig

...

eingeleitet. Die Temperatur stieg im Verlaufe der 5 bis 6-stündigen Umsetzung auf 50°C an. Die abgeschiedenen Kristalle wurden von der Mutterlauge abgetrennt, mit Aceton gewaschen und im Eksikkator getrocknet. Man erhielt 1202 g APTAC. Dies entspricht einer Ausbeute von 97 %, bezogen auf reines DMAPAA.

Beispiel 2

Herstellung von Methacrylamidopropyltrimethylammoniumchlorid

In einem geschlossenen Reaktionsgefäß, das 5000 ml Aceton und 354 g (7Mol) Methylchlorid enthielt, wurden bei einer Temperatur von 50°C im Verlauf von 4 Stunden 1020 g (6 Mol) DMAPMA zugepumpt. Man ließ 4 Stunden nachreagieren, öffnete das Gefäß und arbeitete wie unter 1 auf. Man erhielt 1270 g MAPTAC. Dies entspricht einer Ausbeute von 96 %, bezogen auf reines DMAPMA.

Beispiel 3

Herstellung von Acrylamidoneopentyltrimethylammoniumchlorid

In einem Reaktionskessel wurden 1104 g (6 Mol) TEMAPA und 354 g (7 Mol) Methylchlorid in 5000 ml Aceton vorgelegt. Man erhitzte 4 Stunden auf 80°C. Die Aufarbeitung wurde wie in Beispiel 1 durchgeführt. Man erhielt 1238 g, entsprechend 88 % bezogen auf reines TEMAPA.

Beispiel 4

Herstellung von Acrylamidopropyltrimethylammoniumchlorid

In einem geschlossenen Reaktionsgefäß, das 5000 ml Aceton und 354 g (7 Mol) Methylchlorid enthielt, wurden bei einer Temperatur von 60°C im Verlaufe von 4 Stunden 936 g (6 Mol) DMAPAA zugespeist. Man ließ 4 Stunden bei 60°C nachreagieren und arbeitete wie unter 1 auf. Man erhielt 991 g APTAC. Dies entspricht einer Ausbeute von ca. 80 %.

# P a t e n t a n s p r ü c h e

1. Verfahren zur Herstellung von kristallinen Acryl- und Methacrylamidoalkyltrialkylammoniumchloriden der allgemeinen Formel

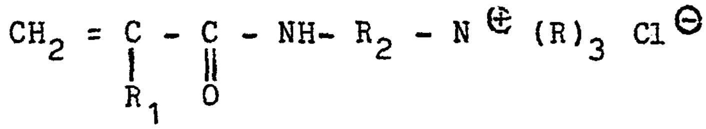

worin R, welches gleich oder verschieden sein kann, eine Alkylgruppe mit 1 bis 3 C-Atomen, $R_1$ Wasserstoff oder die Methylgruppe und $R_2$ die zweiwertige Äthylen-, Propylen- oder 2,2-Dimethylpropylen-Gruppe bedeuten, durch Umsetzung von

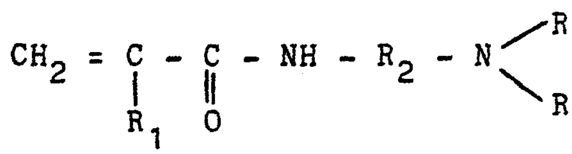

mit RCl,

worin R, $R_1$ und $R_2$ die o.g. Bedeutung besitzen, d a d u r c h g e k e n n z e i c h n e t , daß man die Umsetzung in Aceton, MTBE oder Cyclohexan durchführt und das sich abscheidende kristalline Produkt abfiltriert.

2. Verfahren nach Anspruch 1, d a d u r c h g e k e n n z e i c h n e t , daß man die (Meth)-acrylamid-Ausgangskomponente mit $R = CH_3$ in Aceton löst und Methylchlorid gasförmig einleitet.

3. Verfahren nach Anspruch 1, d a d u r c h g e k e n n z e i c h n e t , daß man in einem geschlossene Gefäß Methylchlorid in Aceton vorlegt und die (Meth)-acrylamid-Ausgangskomponente mit $R = CH_3$ zugibt.

Europäisches Patentamt

# EUROPÄISCHER RECHERCHENBERICHT

0139933

Nummer der Anmeldung

EP 84 10 9441

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 254 929 (CALGON CORP.)<br>* Ansprüche; Seite 50 *<br>----- | 1-3 | C 07 C 103/70 |
| | | | RECHERCHIERTE SACHGEBIETE (Int. Cl.4) |
| | | | C 07 C 103/00 |

Der vorliegende Recherchenbericht wurde für alle Patentansprüche erstellt.

| Recherchenort | Abschlußdatum der Recherche | Prüfer |
|---|---|---|
| DEN HAAG | 29-11-1984 | GAUTIER R.H.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82